Europäisches Patentamt

European Patent Office

Office européen des brevets

(11) Publication number: 0 654 262 A1

# EUROPEAN PATENT APPLICATION

(21) Application number: 94500176.6

(51) Int. Cl.6: **A61K 31/045**, A61K 35/64

(22) Date of filing: 08.11.94

(30) Priority: 09.11.93 CU 10193

(43) Date of publication of application:
24.05.95 Bulletin 95/21

(84) Designated Contracting States:
AT BE CH DE DK ES FR GB GR IE IT LI LU MC
NL PT SE

(71) Applicant: LABORATORIOS DALMER, S.A.
Ave. 25 No. 15819
Municipio Playa,
Ciudad de la Habana (CU)

(72) Inventor: Arruzazabala Valmana, Maria de
Lourdes
Calle H, No. 15010 e/ 7ma. y D,
Reparto Altahabana
Boyeros,
Ciudad de la Habana (CU)
Inventor: Carvajal Ouintana, Daysi
Calle 284 No. 921,
Apto. 5 e/9na B y 11
Reparto Santa Fé,
Ciudad de la Habana (CU)
Inventor: Molina, Sànchez, Vivian
Calle 63 No. 12832 2/128 B y 130
Mariano,
Ciudad de la Habana (CU)
Inventor: Valdes Garcia, Suria
Pasaje Dominguez No. 61,
e/San José y San Luis
Ciudad de la Habana (CU)
Inventor: Diaz Gomez, Maritza
Ayuntamiento No.269 e/Manila y Pellon
Cerro,
Ciudad de la Habana (CU)

(74) Representative: Gil-Vega, Victor
Estébanez Calderon, 3 - 5B
E-28020 Madrid (ES)

(54) A mixture of higher primary aliphatic alcohols, its obtained from beeswax and its pharmaceutical use.

(57) This invention relates to the obtaining of a new natural mixture composed of higher primary aliphatic alcohols, where appropriate with an extended range from 22 to 38 carbon atoms, especially those having between 24 and 34 carbon atoms and more especially those with an unbranched chain of 24, 26, 28, 30, 32 and 34 carbon atoms. This mixture has a relative composition, with respect to each of the alcohols, which is highly reproducible from batch to batch, and is extracted from beeswax.

The said natural mixture has been used efficaciously as an active principle of various pharmaceutical formulations, as anti-ulcer drugs and/or for the protection of the gastric and duodenal mucosa, and also as anti-inflammatories, both topically and orally or parenterally.

EP 0 654 262 A1

This invention relates to the obtaining of a new natural mixture composed of higher primary aliphatic alcohols, where appropriate with an extended range from 22 to 38 carbon atoms, especially those having between 24 and 34 carbon atoms and more especially those with an unbranched chain of 24, 26, 28, 30, 32 and 34 carbon atoms. This natural mixture of alcohols has a relative composition, with respect to each of the alcohols, which is highly reproducible from batch to batch.

The present invention relates essentially to the pharmaceutical industry, and especially to the development of pharmaceutical formulations with specific properties, since the formulations in question may be used against gastric and duodenal ulcers and also as anti-inflammatories.

These formulations contain as active ingredient a new natural mixture composed of higher primary aliphatic alcohols with an unbranched chain of 24 to 34 carbon atoms, especially ones with 24, 26, 28, 30, 32 and 34 carbon atoms, which are obtained from beeswax (hereinafter designated M.H.A.A.B.W.).

Medicinal products with specific pharmacological properties based on the use as active ingredient of these saturated higher primary aliphatic alcohols with an unbranched chain of 24 to 34 carbon atoms have not often been reported; only the case of the natural mixture obtained from sugar-cane waxes (EP-A-0,488,928) has been reported previously.

The use of products produced by bees in empirical medicine has been known since before the present era. With the development of new methods of analysis, it has been possible to identify in many instances the active principle or principles present in the said products, which has enabled some of their biological and pharmacodynamic effects to be elucidated. Among bee-keeping products with therapeutic properties, royal jelly, honey, pollen and beeswax may be mentioned. The latter product is widely used in the pharmaceutical and cosmetics industry for its nutritive, cleansing and other medicinal properties, as well as for use in face packs. Beeswax is essentially composed of the following types of compound:

- saturated and unsaturated, long-chain hydrocarbons (55-75%) in a range from 21 to 37 carbon atoms,
- fatty esters (approximately 30%); these contain long-chain unbranched alcohols (between 16 and 30 carbon atoms),
- free fatty acids (approximately 1-5%) in a range of between 16 and 30 carbon atoms, plus the 18:1 acid which constitutes 30% of this fraction,
- free long-chain alcohols (approximately 1-7%), among which tetracosanol, hexacosanol and triacontanol are the most abundant,
- other, more polar constituents (approximately 1-3%).

The process of the present invention is based on a saponification of previously melted beeswax in a homogeneous phase with concentrated solutions of alkali metal and alkaline-earth metal hydroxides, especially those of low molecular weight and more especially those of sodium, potassium and calcium.

The concentration of the alkaline solution should maintain a weight ratio to the beeswax above 5%, especially from 8 to 25% and more especially between 15 and 25%. The saponification takes place in a time exceeding 30 min, and more especially between 2 and 5 h.

The solid obtained in this step is processed in a conventional solid-liquid extractor, in which the alcohols are selectively extracted employing suitable organic solvents chosen from ketones with 3 to 8 carbon atoms, hydrocarbons having between 6 and 9 carbon atoms, alcohols having between 1 and 5 carbon atoms, haloforms and also aromatic compounds such as benzene and its derivatives, including mixtures of these. Some of the solvents used in the present invention are the following: acetone, methyl ethyl ketone, pentanone, hexanone, tert-butanol, ethanol, methanol, 2-propanol, butanol, hexane, heptane, pentane, octane, chloroform, 1,2-dichloroethane, dichloromethane, trichloroethane, trichloromethane, 1,2,3-trichloropropane, benzene, toluene, phenol, p-methyltoluene and the like.

The extraction takes place in times ranging from 5 to 10 hours. Subsequently, the product obtained is successively recrystallized, using the solvents mentioned above or mixtures thereof. The yield obtained is in the region of 30%, while the purity of the product (M.H.A.A.B.W.) lies in a general range between 80 and 98%, and more especially between 90% and 98%.

The product (M.H.A.A.B.W.) which is obtained in the present invention is a mixture composed of high molecular weight primary aliphatic alcohols having between 24 and 34 carbon atoms. The mixture has a melting point of between 80.0 and 82.5°C. The process for obtaining M.H.A.A.B.W. from beeswax has some advantages over those reported previously. One of these advantages relates to the short period of time in which the product is obtained. Another of the advantages of this invention relates to the yields of M.H.A.A.B.W. which are obtained (around 30% by weight) in comparison with other results described previously. Another of the advantages of the proposed process relates to the purity of the product obtained, which is significantly greater than that in other studies reported previously. Table 1 records the more general qualitative and quantitative composition of M.H.A.A.B.W., while the qualitative and quantitative composition of M.H.A.A.B.W. is presented in Table 2.

Table 1

| More general qualitative and quantitative composition of the M.H.A.A.B.W. obtained | |
|---|---|
| Component | Proportion in the mixture |
| 1-tetracosanol | 9.0 - 15.0% |
| 1-hexacosanol | 12.0 - 18.0% |
| 1-octacosanol | 13.0 - 20.0% |
| 1-triacontanol | 20.0 - 30.0% |
| 1-dotriacontanol | 13.0 - 21.0% |
| 1-tetratriacontanol | 1.5 - 3.5% |

Table 2

| More specific qualitative and quantitative composition of the M.H.A.A.B.W. obtained | |
|---|---|
| Component | Proportion in the mixture |
| 1-tetracosanol | 12.5 +/- 1.0% |
| 1-hexacosanol | 14.5 +/- 1.2% |
| 1-octacosanol | 16.5 +/- 2.0% |
| 1-triacontanol | 24.6 +/- 1.6% |
| 1-dotriacontanol | 16.7 +/- 1.4% |
| 1-tetratriacontanol | 2.3 +/- 0.5% |

The daily dose of M.H.A.A.B.W. to be used for the treatment of the different disorders has been established at between 1 and 100 mg per day, and the most appropriate method of administration is oral in the form of tablets, dragees or capsules. Nevertheless, this drug may also be administered parenterally or topically, bearing in mind the uses recommended in the present invention.

The pharmaceutical formulation used orally contains as active ingredient between 0.5 and 25.0% by weight of M.H.A.A.B.W. This dose is obtained by mixing this M.H.A.A.B.W. with different excipients such as aglutinating agents, disintegrating agents, lubricants, glidants or simply fillers. In this case, lactose, sucrose, maize starch, magnesium stearate, microcrystalline cellulose, croscarmellose sodium, gelatin, hydroxypropylmethylcellulose, titanium dioxide, special talc for tablets, polyethylene glycol, and the like, are included among excipients.

One of the objectives of the present invention is to isolate and purify the natural mixture of high molecular weight primary aliphatic alcohols in the range from 24 to 34 carbon atoms starting from beeswax, specifically the mixture containing the primary alcohols with 24, 26, 28, 30, 32 and 34 carbon atoms.

Another of the objectives of this invention is to use the said natural mixture, at relatively low doses, as a component of pharmaceutical formulations used as drugs against gastric and duodenal ulcers; actually demonstrating that this new M.H.A.A.B.W. significantly reduces gastric ulcers induced by asprin, alcohol, indomethacin and other medicinal products which produce gastric ulcers in patients undergoing treatment. For its part, M.H.A.A.B.W. significantly reduces duodenal ulcers.

Another of the objectives of the present invention is to develop pharmaceutical formulations which contain M.H.A.A.B.W. as active principle, for use as anti-inflammatory drugs administered both topically and orally or parenterally.

Finally, a global assessment of the M.H.A.A.B.W. obtained in the present invention and proposed as active ingredient for medicinal formulations enables it to be asserted that this mixture is very safe and well tolerated, which represents a considerable advantage. This is borne out by the results obtained in acute, subchronic and chronic toxicity tests carried out in rodents and lagomorphs, which have reported an absence of toxicity in relation to the drug. What is more, this M.H.A.A.B.W. has not shown teratogenic activity in rodents, neither has it displayed mutagenic potential in the tests carried out. Side effects have not been detected in subjects treated with the product which is the subject of the present invention.

The objectives of the invention will be described below in detail, reference being made to the examples of implementation, which shall not limit the scope of the invention.

Example 1

1000 g of beeswax are taken and melted at 100-110 °C, 200 g of potassium hydroxide dissolved in 150 mL of water being added. This saponification process is maintained for 30 min with periodic stirring. The M.H.A.A.B.W. is extracted from the solid obtained by means of extraction with heptane in a solid-liquid extraction system. The extract obtained is cooled to room temperature and recrystallized in ethyl methyl ketone. 250 g of the said M.H.A.A.B.W. were obtained with a purity of 93.26%. The melting point of the mixture is 81.0-82.5 °C. Table 3 shows the qualitative and quantitative composition of the M.H.A.A.B.W. obtained.

Table 3

| Qualitative and quantitative composition of the M.H.A.A.B.W. obtained | |
| --- | --- |
| Component | Proportion in the mixture |
| 1-tetracosanol | 13.21 |
| 1-hexacosanol | 15.50 |
| 1-octacosanol | 17.89 |
| 1-triacontanol | 26.03 |
| 1-dotriacontanol | 18.05 |
| 1-tetratriacontanol | 2.58 |

Example 2

12 kg of beeswax previously melted at 90-100 °C are taken, and 300 g of sodium hydroxide dissolved in 200 mL of water are added thereto. The saponification process was maintained for 3 hours with stirring. The M.H.A.A.B.W. is extracted with ethanol for 12 hours in a conventional solid-liquid extraction system. The extract obtained is allowed to cool to room temperature, and the solid which is obtained is recrystallized from methanol. 393 g of the said M.H.A.A.B.W. were obtained with a purity of 93.32%. The melting point of the mixture is 80.5-82.0 °C. Table 4 shows the qualitative and quantitative composition of the M.H.A.A.B.W. obtained.

Table 4

| Qualitative and quantitative composition of the M.H.A.A.B.W. obtained | |
| --- | --- |
| Component | Proportion in the mixture |
| 1-tetracosanol | 13.28 |
| 1-hexacosanol | 15.42 |
| 1-octacosanol | 18.11 |
| 1-triacontanol | 26.10 |
| 1-dotriacontanol | 17.73 |
| 1-tetratriacontanol | 2.68 |

Example 3

50 kg of beeswax are taken and melted at 100-120 °C. 12 kg of calcium hydroxide dissolved in 10 L of water are added immediately. The saponification process was maintained for 3.5 h with stirring. The extraction of M.H.A.A.B.W. takes 12 hours, and chloroform is employed as solvent in a solid-liquid extraction system of appropriate capacity. The product thereby obtained is allowed to cool to room temperature and is subsequently recrystallized with heptane; in all, 14.5 kg of M.H.A.A.B.W. were obtained with a purity of 93.77%. The melting point is 81.5-82.5 °C. Table 5 presents the qualitative and quantitative composition of the M.H.A.A.B.W. obtained.

Table 5

| Qualitative and quantitative composition of the M.H.A.A.B.W. obtained | |
|---|---|
| Component | Proportion in the mixture |
| 1-tetracosanol | 13.48 |
| 1-hexacosanol | 16.12 |
| 1-octacosanol | 17.51 |
| 1-triacontanol | 26.55 |
| 1-dotriacontanol | 17.73 |
| 1-tetratriacontanol | 2.38 |

Example 4

50 kg of beeswax are taken and melted at 100-120°C, and 10.6 kg of sodium hydroxide dissolved in 7.5 L of 1:1 ethanol/water solution are added thereto. The saponification process was maintained for 4.5 h with stirring. Extraction of M.H.A.A.B.W. is carried out for 12 hours, employing 2-propanol as solvent in a solid-liquid extractor. The extract is allowed to cool to room temperature, and the solid which is obtained is recrystallized in a toluene/chloroform (1:1) mixture. 9.3 kg of the said M.H.A.A.B.W. were obtained, which product has a purity of 93.36% determined by means of gas chromatography. The melting point is 80.5-82.0°C; Table 6 shows the qualitative and quantitative composition of the M.H.A.A.B.W. obtained.

Table 6

| Qualitative and quantitative composition of the M.H.A.A.B.W. obtained | |
|---|---|
| Component | Proportion in the mixture |
| 1-tetracosanol | 13.33 |
| 1-hexacosanol | 15.54 |
| 1-octacosanol | 17.02 |
| 1-triacontanol | 27.04 |
| 1-dotriacontanol | 17.83 |
| 1-tetratriacontanol | 2.60 |

Example 5

Two pharmaceutical formulations of the tablet type are developed, in which M.H.A.A.B.W. is used as their active component. The composition of each of these formulations is presented in Table 7. These formulations were developed taking into consideration the physical, chemical and physicochemical properties of the active principle.

Table 7

| Pharmaceutical formulations of the tablet type, using M.H.A.A.B.W. as active principle | | |
|---|---|---|
| Component | Formulation 1 | Formulation 2 |
| | (%) | |
| M.H.A.A.B.W. | 5.0 | 15.0 |
| Lactose | 56.5 | 55.0 |
| Maize starch | 15.0 | 10.0 |
| Gelatin | 2.5 | 2.0 |
| Croscarmellose sodium | 5.0 | 4.0 |
| Sucrose | 5.0 | 4.0 |
| Talc | 2.0 | 2.0 |
| Magnesium stearate | 1.5 | 1.0 |
| Microcrystalline cellulose | 7.5 | 7.0 |

Example 6

Female Sprague-Dawley rats weighing 200-250 g and male Hartley guinea pigs weighing 300 to 400 g were adapted to laboratory conditions over 7 days. The rats were distributed at random in different experimental groups. M.H.A.A.B.W. was administered to one group of animals intraperitoneally (i.p.) after 24 h of fasting, in the form of suspension containing 2% Tween $20/H_2O$, while the controls received only, equivalent volumes of the vehicle (Tween/$H_2O$). These treatments were administered one hour before induction of the ulcer. The ulcer-inducing agents were NaOH (0.2 mol/L), alcohol (60%), ASA (40 mg/kg) and indomethacin (50 mg/kg) (indomethacin was dissolved in 5% bicarbonate), which were administered by gastric intubation.

Induction with alcohol: One hour after the treatment with the vehicle and/or with M.H.A.A.B.W. at 1, 5, 25, 50 and 100 mg/kg, each rat received ethyl alcohol (60%) (1 mL/200 g) by means of gastric intubation. One hour later, the rats were sacrificed and the gastric ulcers were quantified.

Induction with ASA: The guinea pigs were distributed in 4 experimental groups: one control group and three groups treated with M.H.A.A.B.W. (5, 25 and 100 mg/kg), the animals being injected i.p. The control group received equivalent volumes of the vehicle. One hour later, ASA (40 mg/kg) is administered via the gastric route and, after 2 hours, the animals were sacrificed and the process of quantification of gastric ulcers was carried out.

Induction with indomethacin: The rats were distributed at random in 3 experimental groups: one control group and 2 groups treated with M.H.A.A.B.W. at 25 and 50 mg/kg, administered i.p. One hour later, indomethacin (50 mg/kg) was administered by gastric intubation and, after 4 h, the animals were sacrificed, the quantification of gastric ulcers being carried out. In all cases, when ulcers were induced, the stomachs were extracted immediately after sacrifice, opened through the greater curvature and washed in saline solution, and the ulcers were measured. The results are expressed as the sum of the length of the lesions in millimetres, which were always evaluated by at least two independent observers.

In all cases, comparison between treated and control groups was carried out by means of the non-parametric Mann-Whitney U test. The results of the effect of M.H.A.A.B.W. on the ulcers induced with alcohol, ASA and indomethacin are shown in Tables 8, 9 and 10, respectively.

Table 8

| Effect of M.H.A.A.B.W. on ulcers induced with alcohol (60%) | | | |
|---|---|---|---|
| Treatment | Dose (mg/kg) | n | (X +/- SD) |
| | | | Ulcer size (mm) |
| Control | | 25 | 35.46 +/- 4.81 |
| M.H.A.A.B.W. | 1 | 10 | 35.28 +/- 9.13 |
| | 5 | 10 | 4.11 +/- 1.57*** |
| | 25 | 10 | 8.90 +/- 3.82*** |
| | 100 | 10 | 9.35 +/- 3.88** |

**p ≪ 0.01;
*** p ≪ 0.001 (Mann-Whitney U)

Table 9

| Effect of M.H.A.A.B.W. on ulcers induced with ASA (40 mg/kg) | | | |
|---|---|---|---|
| Treatment | Dose (mg/kg) | n | (X +/- SD) |
| | | | Ulcer size (mm) |
| Control | | 8 | 39.37 +/- 10.69 |
| M.H.A.A.B.W. | 5 | 6 | 38.00 +/- 13.90 |
| | 25 | 8 | 9.50 +/- 1.72* |
| | 100 | 6 | 25.83 +/- 3.77 |

*p ≪ 0.05 (Mann-Whitney U)

Table 10

| Effect of M.H.A.A.B.W. on ulcers induced with indomethacin (50 mg/kg) | | | |
|---|---|---|---|
| Treatment | Dose (mg/kg) | n | (X +/- SD) |
| | | | Ulcer size (mm) |
| Control | | 11 | 38.65 +/- 9.48 |
| M.H.A.A.B.W. | 25 | 11 | 17.50 +/- 4.38* |
| | 50 | 10 | 13.14 +/- 3.60* |

* p ≪ 0.05 (Mann-Whitney U)

M.H.A.A.B.W. protected the gastric mucosa in the models of alcohol-, ASA- and indomethacin-induced ulcers (Tables 8, 9 and 10), significantly decreasing the size of the ulcers.

Example 7

The objective of this study is to determine whether the protective effect of M.H.A.A.B.W. on ulcers is prostaglandin-dependent.

Male Sprague-Dawley rats weighing from 150 to 200 g were adapted to laboratory conditions over 7 days, with free access to water and food until 48 h before the experiment, when it is withdrawn. The animals were distributed at random in 4 experimental groups.

M.H.A.A.B.W. was administered i.p. in a suspension in Tween 20/$H_2O$ (2%), and cimetidine was dissolved in hot water and indomethacin in 5% sodium bicarbonate. The 4 experimental groups were as follows (n = 10): 1) control (vehicle) 2 and 3) M.H.A.A.B.W. at 25 and 50 mg/kg, respectively, and 4) cimetidine 25 mg/kg. Immediately after the animals were injected intraperitoneally, indomethacine was administered to them subcutaneously at a dose of 10 mg/kg. Half an hour later, 60% ethanol was administered to them orally, and the animals were sacrificed after a further half hour. The animals' stomachs were extracted and opened through the greater curvature, and the ulcers were quantified by observation using a 3 x magnification. The results are expressed in millimetres.

Comparison of the control group and treated animals was carried out by means of the non-parametric Mann-Whitney U test. The results of the effect of M.H.A.A.B.W. on alcohol- and indomethacin-induced ulcer are shown in Table 11.

Table 11

| Effect of M.H.A.A.B.W. on ulcer induced with alcohol (60%) and indomethacin (10 mg/kg) | | | | |
|---|---|---|---|---|
| Treatment | Dose (mg/-kg) | n | Ulcers (mm) | Inhibition (%) |
| 60% alcohol<br>Indomethacin<br>Indomethacin + alcohol + Tween | | 10<br>10<br>9 | 35.3 + 2.95<br>0<br>54.6 +/- 8.55 | |
| M.H.A.A.B.W. | 25<br>50 | 8<br>5 | 13.8 +/- 5.44*<br>18.0 +/- 6.04* | 72.8<br>64.5 |
| Cimetidine | 25 | 9 | 14.6 +/- 5.64* | 71.2 |

* p ≪ 0.05 Mann-Whitney U

As observed in the said table, when either M.H.A.A.B.W. or cimetidine was used, alcohol-induced ulcers were decreased at the doses evaluated, even with the prior administration of indomethacin. The latter on its own did not produce ulcers and, moreover, potentiated the ulcerous effect of alcohol.

The experimental design is based on the administration of indomethacin prior to the induction of the ulcer with alcohol, so that, if the compound maintains its protective effect with respect to the ulcer, this effect will be prostaglandin-independent; this indicates that the anti-ulcer effect of M.H.A.A.B.W. is maintained despite having administered indomethacin, and we can accordingly put forward the view that the said protector effect could be prostaglandin-independent.

Example 8

The results obtained in the previous examples suggest an action on ulcers induced by both acid-dependent and -independent compounds. With the aim of ruling out an effect on the inhibition of gastric acid secretion, the action of M.H.A.A.B.W. on gastric acid secretion was determined in a model of ligation of the pylorus.

Female Sprague-Dawley rats weighing between 150 and 200 g were adapted over 15 days to laboratory conditions with free access to water and food. The animals were distributed at random in different experimental groups and were deprived of food during the 48 hours preceding ligation. M.H.A.A.B.W. was administered i.p. in the form of a suspension in 2% Tween 20.

Cimetidine was dissolved in hot water and administered in a manner similar to M.H.A.A.B.W.. The animals were distributed in 6 experimental groups: 1) control (vehicle); 2, 3 and 4) M.H.A.A.B.W. at 25, 50 and 100 mg/kg, respectively; 5 and 6) (positive control) cimetidine at 25 and 50 mg/kg, respectively.

The rats were anaesthetized with ether and an incision was made in the abdomen, the stomach was extracted and the pylorus was ligated with a suture thread. Immediately afterwards, the compound was administered i.p. and 3 mL of saline solution were injected subcutaneously. After 4 h, the animals were sacrificed, and the intact stomach clamped at the oesophagus was extracted. The gastric contents were centrifuged at 3000 rpm for 10 minutes and their volume was subsequently measured. The acidity of the gastric juice was assessed by means of titration with 0.1 mol/L NaOH, employing phenolphthalein as indicator. Comparison between the treated groups and the control group was carried out by means of the non-parametric Mann-Whitney U test. The results for M.H.A.A.B.W. on gastric acidity with ligation of the pylorus are shown in Table 12.

Table 12

| Effect of M.H.A.A.B.W. on gastric acidity in the model of pyloric ligation | | | | |
|---|---|---|---|---|
| Treatment | Dose (mg/kg) | n | Volume gastric juice (mL) | Acidity (meq $H^+$/mL) |
| Control | | 12 | 8.18 +/- 0.51 | 0.109 +/- 0.002 |
| M.H.A.A.B.W. | 25 | 10 | 5.00 +/- 0.58* | 0.09 +/- 0.01 |
| | 50 | 11 | 5.51 +/- 0.54* | 0.104 +/- 0.006 |
| | 100 | 10 | 5.49 +/- 0.65* | 0.114 +/- 0.002 |
| Cimetidine | 25 | 9 | 5.87 +/- 0.63* | 0.080 +/- 0.009* |
| | 50 | 9 | 5.83 +/- 0.38** | 0.083 +/- 0.006** |

*$p \ll 0.05$;

**$p \ll 0.01$ Mann-Whitney U

As is seen, on administering M.H.A.A.B.W. (25, 50 and 100 mg/kg), the volume of gastric juice in rats with pyloric ligation was significantly reduced, the maximum effect being obtained at the smallest dose employed (25 mg/kg). Cimetidine at 25 and 50 mg/kg significantly reduced both the volume of gastric juice and the $H^+$ ion concentration.

In this study, it is demonstrated that M.H.A.A.B.W. (25, 50 and 100 mg/kg) significantly inhibits the volume of gastric juice in the model of ligation of the pylorus without affecting the $H^+$ content, a finding which virtually rules out an inhibitory effect on $H^+$ ion secretion of the cimetidine or omeprazole type.

Example 9

To verify the anti-inflammatory effect of M.H.A.A.B.W., the models of carrageenan-induced pleurisy and cotton-induced granuloma are employed, which constitute established models for the evaluation of anti-inflammatory drugs.

Cotton-induced granuloma: Male Sprague-Dawley rats weighing between 200 and 250 g were adapted to laboratory conditions in the same manner as in the previous experiment. M.H.A.A.B.W. was administered orally in the form of a suspension in Tween 20/$H_2O$ (2%), while indomethacin was dissolved in 5% bicarbonate. Both treatments were administered by means of gastric intubation during the 6 days subsequent to the implantation of the cotton. The rats were distributed at random in 5 experimental groups: 1) control (vehicle), 2, 3 and 4) M.H.A.A.B.W. at 25, 50 and 100 mg/kg, respectively, and 5) indomethacin at 3 mg/kg. The rats were previously anaesthetized with ether, an incision being made in their dorsolateral midline. The sterile cotton pellet weighing 50 mg is positioned subcutaneously, applying a local antiseptic, and the wound is finally sutured.

One day after the final administration, the animals were sacrificed in an ether atmosphere and the granulomas were carefully dissected and placed in an oven at 60°C for 24 h, their dry weights being determined. The weight of the cotton pellet (50 mg) was subtracted from the weight of the granuloma. The percentage inhibition was expressed by taking the weight of the granuloma formed in the control group as reference. The results for the effect of M.H.A.A.B.W. on cotton-induced granuloma are shown in Table 13.

Table 13

| Effect of the administration of M.H.A.A.B.W. on the model of cotton-induced granuloma. | | | | |
|---|---|---|---|---|
| Treatment | Dose (mg/kg) | n | Weight of the granuloma (mg) | Inhibition (%) |
| Control | | 8 | 258 + 0.12 | |
| M.H.A.A.B.W. | 25 | 8 | 138 + 0.05* | 46.51 |
| | 50 | 7 | 162 + 0.11** | 54.7 |
| | 100 | 9 | 117 + 0.04** | 54.7 |
| Indomethacin | 3 | 5 | 79 + 0.02** | 69.3 |

*p< 0.05,
** p< 0.01 Mann-Whitney U

As seen in the table, M.H.A.A.B.W. significantly reduced the weight of the granuloma when administered in a range from 25 to 100 mg/kg, the maximum response being obtained at the lowest dose employed (25 mg/kg). However, at none of the doses used is a 100% reduction in inflammation observed.

Carrageenan-induced pleurisy: Male Sprague-Dawley rats (200-250 g) were used, which were maintained under laboratory conditions with free access to water and food for 15 days, and were deprived of food during the 12 h before the experiment. M.H.A.A.B.W. was administered in the form of a suspension in a gum acacia/$H_2O$ (10 mg/mL) vehicle, as was triacontanol, while indomethacin was dissolved in 5% sodium bicarbonate.

All the treatments were administered by means of gastric intubation for one hour prior to the injection of carrageenan. The rats were distributed at random in the following experimental groups: 1) control (vehicle); 2, 3 and 4) M.H.A.A.B.W. at 25, 50 and 100 mg/kg, respectively; 5) triacontanol at 50 mg/kg; and 6) indomethacin at 10 mg/kg.

For the induction of the oedema, the rats were anaesthetized with ether, and 0.3 mL of 1% carrageenan in saline solution was injected into the pleural cavity. Five hours later, the animals were sacrificed and the exudate was collected and its volume measured. The percentage inhibition of the oedema was calculated by means of the formula:

Inhibition (%) = 1 - (VT)/(VC) x 100

where:
VT = oedema volume in the treated animals
VC = oedema volume in the control animals
The results obtained are shown in Table 14.

Table 14

| Effect of the oral administration of M.H.A.A.B.W. in carrageenan-induced pleurisy | | | | |
|---|---|---|---|---|
| Treatment | Dose (mg/kg) | n | Exudate volume (mL) | Inhibition (%) |
| Control | | 15 | 1.24 + 0.30 | |
| M.H.A.A.B.W. | 25 | 10 | 1.13 + 0.39 | 8.9 |
| | 50 | 10 | 0.98 + 0.36 | 20.9 |
| | 100 | 11 | 0.85 + 0.28* | 31.4 |
| Triacontanol | 100 | 10 | 0.96 + 0.30* | 27.4 |
| Indomethacin | 10 | 14 | 0.50 + 0.15*** | 59.6 |

* p< 0.05,
*** p<0.001 Mann-Whitney U

As seen in the table, M.H.A.A.B.W. at the maximum dose tested of 100 mg/kg showed a moderate anti-inflammatory effect, inhibiting the volume of pleural exudate by 23.3%, this being the maximum effect observed, while indomethacin (10 mg/kg) produced an effective protection with respect to the oedema of a 59.6% inhibition. Triacontanol also proved effective, but its effectiveness proves less than that shown by M.H.A.A.B.W. at similar doses.

These results show that M.H.A.A.B.W. is more effective in the model of cotton-induced granuloma than in carrageenan-induced pleurisy. These two experimental models differ in various aspects: for example, in cotton-induced granuloma, cell migration (neutrophil leukocytes) is the most significant feature, while in carrageenan-induced pleurisy the inflammatory exudate (oedema through increase in vascular permeability) is most significant, this latter effect being very dependent on prostaglandins (E and F). Of the mediators of inflammation, the leukotrienes, most especially $LTB_4$, have a potent chemotactic effect on polymorphonuclear leukocytes, inducing aggregation, degranulation and release of lysosomal enzymes from the latter.

A possible explanation of this finding is that M.H.A.A.B.W. might inhibit, at some point, the synthesis or release of leukotrienes, causing an anti-inflammatory action (inhibition of cell migration), but might also shift A.A. metabolism towards the formation of primary prostaglandins which mediate an increase in the exudate fluid. Consequently, M.H.A.A.B.W. would be effective as anti-inflammatory only in those processes in which cell migration was more actively involved.

Example 10

A group of albino guinea pigs of both sexes weighing on average between 280-350 g was maintained under laboratory conditions for one week with free access to water and food. These animals were anaesthetized with pentobarbitone sodium (50 mg/kg) i.p. A cannula was inserted into the trachea and connected to a ventilation pump (55 breaths/min). The animals were ventilated artificially with a volume of 3-4 mL. Tracheal pressure was measured using a pressure transducer (MP 0.5) connected to a polygraph.

The M.H.A.A.B.W. which is the subject of the present invention was administered intravenously prior to the administration of PAF (40 ng/kg, i.p.) at doses of 10.0, 1.0 and 0.5 mg/kg, respectively. To determine the effect of the said M.H.A.A.B.W. on the response to histamine induced by PAF, the animals were treated 10 min before the administration of M.H.A.A.B.W. with PAF or with saline solution (controls) after reproducible responses to histamine (2-12 $\mu$g/kg i.v.) were obtained in all cases. The results obtained are shown in Tables 15 and 16.

Table 15

| Effect of M.H.A.A.B.W. on PAF-induced bronchoconstriction in guinea pigs | | |
|---|---|---|
| | Dose (mg/kg) | % maximum bronchoconstriction |
| Controls | -- | 39.60 |
| M.H.A.A.B.W. | 1.0 | 13.80* |
| Controls | -- | 21.28 |
| M.H.A.A.B.W. | 0.5 | 20.26 n.s. |

* $p < 0.05$ (Mann-Whitney U test)

The percentage maximum bronchoconstriction was obtained by occluding the trachea of each animal at the end of each experiment.

Table 16

| Effect of M.H.A.A.B.W. on the hyper-response to histamine induced by PAF (40 ng/kg) in guinea pigs | | | | |
|---|---|---|---|---|
| | Dose (mg/kg) | n | Bronchoconstriction (%) | |
| | | | pre-PAF | post-PAF |
| Controls | -- | 8 | 100 | 157 +/- 28 |
| M.H.A.A.B.W. | 10 | 6 | 100 | 105 +/- 13** |
| Controls | -- | 5 | 100 | 199 +/- 47.6 |
| M.H.A.A.B.W. | 1.0 | 5 | 100 | 117 +/- 25.3** |
| Controls | -- | 4 | 100 | 183.25 +/- 18.0 |
| M.H.A.A.B.W. | 0.5 | 4 | 100 | 172.5 +/- 15 n.s. |

** $p < 0.01$ (Mann-Whitney U test)

The response to histamine before, the administration of PAF was taken as 100% in each of the animals. M.H.A.A.B.W. caused changes in the bronchoconstrictor response to histamine; however, it significantly decreased both PAF-induced bronchoconstriction and the hyper-response to histamine induced by this agent at concentrations of between 1 and 10 mg/kg.

This M.H.A.A.B.W. possesses an effect on receptors for PAF and/or leukotriene antagonists and/or lipoxygenase inhibitors. This effect might explain the anti-ulcer and anti-inflammatory action previously reported. As may be seen, the new M.H.A.A.B.W. which is the subject of the present invention neither blocked nor potentiated the bronchoconstrictor effect of histamine, so that the possibility of an antihistaminic effect is ruled out.

Example 11

A comparative study is carried out of the properties of the natural mixtures of high molecular weight primary aliphatic alcohols obtained from beeswax (M.H.A.A.B.W.) which are the subject of the present invention, and the mixture of high molecular weight higher aliphatic alcohols obtained from sugar-cane wax (hereinafter designated M.H.A.A.S.C.W.). The said study enables it to be established that not only do these differ in the relative composition of alcohols they possess, but also that their pharmacological profiles in various experimental models traditionally used in pharmacological screening also differ. To this end, the experiments which will be described briefly below are carried out:

a) anti-inflammatory effect: To corroborate the anti-inflammatory effect of the two mixtures, the models of carrageenan-induced pleurisy and cotton-induced granuloma were employed, using doses of 100 and 200 mg/kg, respectively.

b) anti-ulcer effect: To corroborate the anti-ulcer effect of the two mixtures, the experimental methodology described in Example 8 of the present invention was followed, using a dose of 25 mg/kg body weight for each of the mixtures.

c) hypolipaemic effect: Male New Zealand rabbits were taken and divided into the following groups: a) control; b) M.H.A.A.B.W. (5 mg/kg); c) M.H.A.A.S.C.W. (5 mg/kg), administered orally for 1 month. Every 15 days, blood samples were taken from them to determine the parameters of lipid metabolism (total cholesterol, triglycerides, HDL-C, LDL-C and VLDL-C).

d) anti-ischaemic effect: For analysis of the effect of the two mixtures on cerebral ischaemia, the model of provocation of cerebral ischaemia in Mongolian gerbils by carotid ligation was used. Female Mongolian gerbils weighing 60 and 80 g were used, which were adapted to laboratory conditions with free access to water and food. Both mixtures were administered intraperitoneally (i.p.), using a suspension in a 2% Tween 20/$H_2O$ vehicle for this purpose. The animals were distributed in the following experimental groups: 1) control (Tween 20/$H_2O$ vehicle), 2) M.H.A.A.B.W. 200 mg/kg and 3) M.H.A.A.S.C.W. 200 mg/kg.

Ligation of the left common carotid was carried out, anaesthetizing the animals in an ether atmosphere. The animals were observed for 24 h, recording the appearance of clinical symptoms of cerebral damage, such as circling, rolling and convulsions, as well as the number of deaths occurring within the experiment.

e) platelet aggregation-inhibitory effect: To corroborate the effect of the two mixtures on platelet aggregation in rats induced with ADP and collagen, a group of male Sprague-Dawley rats weighing 250-350 g was used, the animals being distributed at random in 3 experimental groups. Each of the mixtures was administered for 4 weeks by means of gastric intubation, in the form of a suspension in a gum acacia-water vehicle (1 mL/100 g body weight), to the following experimental groups: a) control (to which only the vehicle was administered); b) M.H.A.A.B.W. (25 mg/kg) and c) M.H.A.A.S.C.W. (25 mg/kg).

For the platelet aggregation test, the rats were anaesthetized in an ether atmosphere. After opening of their abdomen, blood (5 mL) was extracted from the vena cava and mixed with 3.8% sodium citrate (1 volume of citrate to 9 volumes of blood). Platelet-rich plasma (PRP) was obtained by centrifugation of the blood, and platelet-poor plasma (PPP) was obtained by centrifugation of aliquots of PRP at 330 g for 15 min. Platelet aggregation was induced with ADP and with collagen, and was recorded in a Payton aggregometer.

f) antithrombotic effect: To study the antithrombotic effect, the venous thrombosis model was employed. To this end the following treatments were administered: 1) Control, 2, 3, 4) M.H.A.A.B.W. (25, 50 and 100 mg/kg), respectively, and 5, 6, 7) M.H.A.A.S.C.W. (25, 50 and 100 mg/kg), respectively.

The rats were anaesthetized with pentobarbitone sodium (40 mg/kg) i.p. Subsequently, they were injected with hypotonic saline solution (0.22% NaCl) (1 mL/100 g weight) via the femoral vein. One minute later, the abdominal cavity was opened and the vena cava exposed, isolated and ligated by passing a thread through it. The abdominal cavity was closed temporarily for 10 min and afterwards reopened, and the vena cava was ligated again 2 cm below the first ligature, the piece being extracted immediately and opened longitudinally. The thrombus was then extracted, placed in a moist chamber at room temperature and weighed one hour later.

The results obtained when all these pharmacological tests were carried out are presented in summary form in Table 17.

Table 17

| Comparative effect of the natural mixture of alcohols obtained from beeswax and of that obtained from sugar-cane wax | | |
|---|---|---|
| Test evaluated | M.H.A.A.B.W. | M.H.A.A.S.C.W. |
| anti-inflammatory | + | - |
| hypolipaemic | - | + + + |
| anti-ischaemic | + | + + + |
| anti-ulcer | + + + | + |
| platelet aggregation-inhibitory | - | + |
| antithrombotic | - | + |
| displays:<br>( + ) mild activity<br>( + + ) moderate activity<br>( + + + ) high activity<br>(-) no activity | | |

As seen from these results, the biological properties of the two natural mixtures of high molecular weight primary aliphatic alcohols are different; only in the anti-ulcer effect do both mixtures display activity, although, as may be seen in Table 17, the effect of the mixture of alcohols obtained from beeswax (M.H.A.A.B.W.) is much more effective than that of the mixture of alcohols obtained from sugar-cane wax (M.H.A.A.S.C.W.).

**Claims**

1. Pharmaceutical composition for the treatment of gastric and duodenal ulcers which also displays anti-inflammatory activity, which contains as active ingredient a mixture of higher primary alcohols having between 24 and 34 carbon atoms, whose more general composition is the following:

| 1-tetracosanol | 9.0 - 15.0% |
|---|---|
| 1-hexacosanol | 12.0 - 18.0% |
| 1-octacosanol | 13.0 - 20.0% |
| 1-triacontanol | 20.0 - 30.0% |
| 1-dotriacontanol | 13.0 - 21.0% |
| 1-tetratriacontanol | 1.5 - 3.5% |

2. Pharmaceutical composition according to Claim 1, for the treatment of gastric and duodenal ulcers which also displays anti-inflammatory activity, which contains as active ingredient a mixture of higher primary alcohols having between 24 and 34 carbon atoms, with the following more specific composition:

| 1-tetracosanol | 12.5 +/- 1.0% |
|---|---|
| 1-hexacosanol | 14.5 +/- 1.2% |
| 1-octacosanol | 16.5 +/- 2.0% |
| 1-triacontanol | 24.6 +/- 1.6% |
| 1-dotriacontanol | 16.7 +/- 1.4% |
| 1-tetratriacontanol | 2.3 +/- 0.5% |

3. Method for obtaining a natural mixture composed of high molecular weight primary aliphatic alcohols, obtained from beeswax, according to Claims 1 and 2, characterized in that beeswax is melted, saponification preferably takes place in a homogeneous phase with solutions of alkali metal and alkaline-earth metal hydroxides, and extraction of the natural mixture is preferably carried out in a solid-liquid extraction system using as organic solvents hydrocarbons with 6 to 9 carbon atoms, ketones with 3 to 8 carbon atoms, alcohols with 1 to 5 carbon atoms, haloforms and aromatic compounds, as well as mixtures of these. Optionally, the natural mixture is recrystallized in the solvents mentioned above and mixtures thereof.

4. Extraction method according to Claim 3, characterized in that the temperature of melting of the beeswax lies between 90 and 150°C, the hydroxide concentration lies in the range from 5 to 30%, the saponification time range begins from 30 min and extends upwards and the extraction time varies from 1 to 20 hours.

5. Extraction method according to Claims 3 and 4, characterized in that the hydroxides used in the saponification of the beeswax are those of sodium, calcium and potassium.

6. Extraction method according to Claims 3 and 4, characterized in that pentane, hexane, heptane and octane are used as hydrocarbons in the extraction process.

7. Extraction method according to Claims 3 and 4, characterized in that acetone, pentanone, methyl ethyl ketone, methyl butyl ketone and 3-heptanone are used as ketones in the extraction.

8. Extraction method according to Claims 3 and 4, characterized in that methanol, ethanol, n-propanol, 2-propanol, n-butanol, 2-butanol, n-pentanol and tert-butanol are used as alcohols in the extraction.

9. Extraction method according to Claims 3 and 4, characterized in that benzene, toluene, ethylbenzene, phenol and p-methyltoluene are used as aromatic solvents in the extraction.

10. Extraction method according to Claims 3 and 4, characterized in that dichloromethane, 1,2-dichloroethane, chloroform, trichloroethane, 1,2-dichloropropane and 1,2,3-trichloropropane are used as haloforms in the extraction process.

11. Pharmaceutical formulations such as tablets, capsules or dragrées, characterized in that they contain as active principle a natural mixture of high molecular weight primary aliphatic alcohols, obtained from beeswax, according to Claims 1 and 2, in a proportion of 0.5 to 25.0% by weight of the active ingredient, as well as fillers, aglutinating agents, disintegrating agents, lubricants and other pharmaceutical excipients.

12. Use of the natural mixture of high molecular weight primary aliphatic alcohols obtained from beeswax according to Claims 1 and 2, or pharmaceutical formulations according to Claim 11, for the treatment of gastric and duodenal ulcers.

13. Use of the natural mixture of high molecular weight primary aliphatic alcohols, obtained from beeswax, according to Claims 1 and 2, or pharmaceutical formulations according to Claim 11, as an anti-inflammatory agent.

14. Use of the natural mixture of high molecular weight primary aliphatic alcohols, obtained from beeswax, according to Claims 1 and 2, or pharmaceutical formulations according to Claim 11, in a daily dose of 1 to 100 mg of this natural mixture of alcohols, especially of 10 to 20 mg administered orally or parenterally.

**PARTIAL EUROPEAN SEARCH REPORT** Application Number

which under Rule 45 of the European Patent Convention EP 94 50 0176
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 046 995 (EISAI CO., LTD) 10 March 1982 <br> * abstract * | 1,2 | A61K31/045 <br> A61K35/64 |
| X,P | WO-A-94 07830 (LABORATORIOS DALMER SA) 14 April 1994 <br> * the whole document * | 3-10 | |

**TECHNICAL FIELDS SEARCHED** (Int.Cl.6)

A61K

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 13 February 1995 | Leherte, C |

EPO FORM 1503 03.82 (P04C07)

EP 94 50 0176

-C-

INCOMPLETE SEARCH

Claims not searched: 11-14

Method for treatment of the human or
animal body by surgery or therapy
(see article 52(4) of the European
Patent Convention)